(19) Europäisches Patentamt
European Patent Office
Office européen des brevets

(11) **EP 1 002 519 B1**

(12) **FASCICULE DE BREVET EUROPEEN**

(45) Date de publication et mention de la délivrance du brevet:
**14.04.2004 Bulletin 2004/16**

(51) Int Cl.$^7$: **A61K 7/13**

(21) Numéro de dépôt: **99402647.4**

(22) Date de dépôt: **25.10.1999**

(54) **Composition de teiture d'oxydation des fibres kératiniques et procédé de teinture mettant en oeuvre cette composition**

Oxidationsfärbemittel für keratinische Fäsern und Färbungsverfahren mit diesem Mittel

Composition for oxidative dyeing of keratinous fibres and dyeing process using same

(84) Etats contractants désignés:
**AT BE CH CY DE DK ES FI FR GB GR IE IT LI LU MC NL PT SE**

(30) Priorité: **20.11.1998 FR 9814652**

(43) Date de publication de la demande:
**24.05.2000 Bulletin 2000/21**

(73) Titulaire: **L'OREAL**
**75008 Paris (FR)**

(72) Inventeur: **Audousset, Marie-Pascale**
**92600 Asnières (FR)**

(74) Mandataire: **Goulard, Sophie et al**
**L'OREAL-DPI**
**6 rue Bertrand Sincholle**
**92585 Clichy Cedex (FR)**

(56) Documents cités:
**DE-A- 19 637 371**
**DE-C- 19 534 214**
**GB-A- 2 260 135**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

L'invention a pour objet une composition pour la teinture d'oxydation des fibres kératiniques, et en particulier des fibres kératiniques humaines telles que les cheveux comprenant, dans un milieu approprié pour la teinture, au moins une base d'oxydation, du 1,3-bis-(β-hydroxyéthyl)amino 2-méthyl benzène et/ou au moins l'un de ses sels d'addition avec un acide à titre de premier coupleur, et au moins un méta-aminophénol substitué sélectionné à titre de second coupleur, ainsi que le procédé de teinture mettant en oeuvre cette composition.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des bases hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans les demandes de brevet FR-A-2 681 860 et DE-A-19 637 371, des compositions pour la teinture d'oxydation des fibres kératiniques contenant une ou plusieurs bases d'oxydation, un ou plusieurs dérivés de 2,6-diamino toluène à titre de coupleur, et éventuellement un ou plusieurs coupleurs additionnels choisis parmi les coupleurs classiquement utilisés dans le domaine de la coloration d'oxydation, tels que le méta-aminophénol et le 2-méthyl 5-amino phénol. Cependant bien que les colorations obtenues en mettant en oeuvre de telles compositions soient très chromatiques, elles ne sont pas entièrement satisfaisantes, notamment du point de vue de leur sélectivité et de leur ténacité vis à vis des différents traitements et agressions naturelles que peuvent subir les fibres kératiniques.

Or, la demanderesse vient maintenant de découvrir qu'il est possible d'obtenir de nouvelles teintures, capables de conduire à des colorations puissantes et très chromatiques, et résistant bien aux diverses agressions que peuvent subir les fibres, en associant au moins une base d'oxydation, du 1,3-bis-(β-hydroxyéthyl) amino 2-méthyl benzène et/ou au moins l'un de ses sels d'addition avec un acide à titre de premier coupleur, et au moins un méta-aminophénol substitué convenablement sélectionné à titre de second coupleur.

Cette découverte est à la base de la présente invention.

L'invention a donc pour premier objet une composition pour la teinture d'oxydation des fibres kératiniques et en particulier des fibres kératiniques humaines telles que les cheveux, caractérisée par le fait qu'elle comprend, dans un milieu approprié pour la teinture :

- au moins une base d'oxydation,
- du 1,3-bis-(β-hydroxyéthyl)amino 2-méthyl benzène et/ou au moins l'un de ses sels d'addition avec un acide à titre de premier coupleur ;
- et au moins un second coupleur choisi parmi les méta-aminophénols substitués de formules (I) et (I bis) ci-après, et leurs sels d'addition avec un acide :

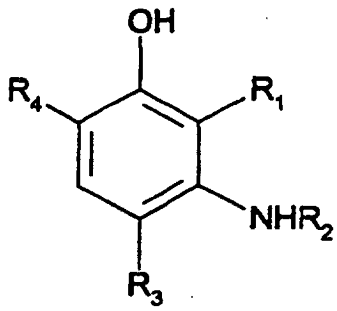 (I) 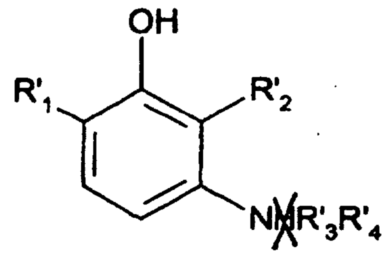 (I bis)

dans lesquelles

- $R_1$ représente un atome d'hydrogène, un radical alkyle en $C_1$-$C_4$, monohydroxyalkyle en $C_1$-$C_4$, ou polyhydroxyalkyle en $C_2$-$C_4$ ;
- $R_2$ représente un atome d'hydrogène, un radical alkyle en $C_1$-$C_4$, monohydroxyalkyle en $C_1$-$C_4$ ou polyhydroxyalkyle en $C_2$-$C_4$ ;
- $R_3$ représente un atome d'hydrogène, un radical alkyle en $C_1$-$C_4$, alcoxy en $C_1$-$C_4$ ou un atome d'halogène choisi parmi le chlore, le brome, l'iode ou le fluor ;
- $R_4$ représente un atome d'hydrogène, un radical alkyle en $C_1$-$C_4$, alcoxy en $C_1$-$C_4$, monohydroxyalkyle en $C_1$-$C_4$, polyhydroxyalkyle en $C_2$-$C_4$, monohydroxyalcoxy en $C_1$-$C_4$ ou polyhydroxyalcoxy en $C_2$-$C_4$ ;
- $R'_1$ et $R'_2$, identiques ou différents, représentent un atome d'hydrogène ou d'halogène choisi parmi le chlore, le brome, l'iode ou le fluor, un radical alkyle en $C_1$-$C_4$, monohydroxyalkyle en $C_1$-$C_4$, polyhydroxyalkyle en $C_2$-$C_4$, alcoxy en $C_1$-$C_4$, monohydroxyalcoxy en $C_1$-$C_4$ ou polyhydroxyalcoxy en $C_2$-$C_4$ ;
- $R'_3$ et $R'_4$, identiques ou différents, représentent un atome d'hydrogène, un radical alkyle en $C_1$-$C_4$, monohydroxyalkyle en $C_1$-$C_4$, polyhydroxyalkyle en $C_2$-$C_4$ ou monoaminoalkyle en $C_1$-$C_4$ ;

étant entendu que :

- au moins un des radicaux $R_1$ à $R_4$ est différent d'un atome d'hydrogène ;
- au moins un des radicaux $R_1$, $R_3$ et $R_4$ représente un atome d'hydrogène ;
- au moins un des radicaux $R'_1$ et $R'_2$ représente un atome d'halogène ;
- et lorsque que $R_1$, $R_2$ et $R_3$ représentent simultanément un atome d'hydrogène, alors $R_4$ est différent d'un radical méthyle.

La composition tinctoriale conforme à l'invention conduit à des colorations puissantes, très chromatiques, et présentant d'excellentes propriétés de résistances à la fois vis à vis des agents atmosphériques tels que la lumière et les intempéries et vis à vis de la transpiration et des différents traitements que peuvent subir les cheveux. Ces propriétés sont particulièrement remarquables, notamment en ce qui concerne la sélectivité et la résistance des colorations vis à vis de l'action de la permanente.

L'invention a également pour objet un procédé de teinture d'oxydation des fibres kératiniques mettant en oeuvre cette composition tinctoriale.

Parmi les méta-aminophénols substitués de formules (I) et (I bis) ci-dessus on peut plus particulièrement citer le 5-N-(β-hydroxyéthyl)amino 2-méthyl phénol, le 3-amino 2-chloro 6-méthyl phénol, le 5-amino 2-méthoxy phénol, le 5-amino 2-(β-hydroxyéthyloxy) phénol, le 5-N-(β-hydroxyéthyl)amino 4-méthoxy 2-méthyl phénol, le 5-amino 4-méthoxy 2-méthyl phénol, le 5-amino 4-chloro 2-méthyl phénol, le 5-amino 2,4-diméthoxy phénol, le 5-(γ-hydroxypropylamino) 2-méthyl phénol, le 3-amino 6-chloro phénol, le 3-amino 6-bromo phénol, le 3-(β-aminoéthyl)amino 6-chloro phénol, le 3-(β-hydroxyéthyl)amino 6-chloro phénol, et leurs sels d'addition avec un acide.

La nature de la ou des bases d'oxydation utilisées dans la composition tinctoriale prête à l'emploi n'est pas critique. Elles peuvent notamment être choisies parmi les paraphénylènediamines, les bases doubles, les para-aminophénols, les ortho aminophénols et les bases d'oxydation hétérocycliques.

Parmi les paraphénylénediamines utilisables à titre de base d'oxydation dans les composition tinctoriales conformes à l'invention, on peut notamment citer les composés de formule (II) suivante et leurs sels d'addition avec un acide :

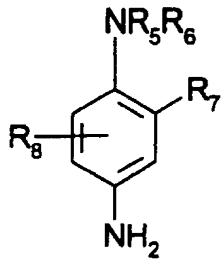

(II)

dans laquelle :

- $R_5$ représente un atome d'hydrogène, un radical alkyle en $C_1$-$C_4$, monohydroxyalkyle en $C_1$-$C_4$, polyhydroxyalkyle en $C_2$-$C_4$, alcoxy($C_1$-$C_4$)alkyle($C_1$-$C_4$), alkyle en $C_1$-$C_4$ substitué par un groupement azoté, phényle ou 4'-aminophényle ;
- $R_6$ représente un atome d'hydrogène, un radical alkyle en $C_1$-$C_4$, monohydroxyalkyle en $C_1$-$C_4$, polyhydroxyalkyle en $C_2$-$C_4$, alcoxy($C_1$-$C_4$)alkyle($C_1$-$C_4$) ou alkyle en $C_1$-$C_4$ substitué par un groupement azoté ;
- $R_7$ représente un atome d'hydrogène, un atome d'halogène tel qu'un atome de chlore, de brome, d'iode ou de fluor, un radical alkyle en $C_1$-$C_4$, monohydroxyalkyle en $C_1$-$C_4$, hydroxyalcoxy en $C_1$-$C_4$, acétylaminoalcoxy en $C_1$-$C_4$, mésylaminoalcoxy en $C_1$-$C_4$ ou carbamoylaminoalcoxy en $C_1$-$C_4$ ;
- $R_8$ représente un atome d'hydrogène, d'halogène ou un radical alkyle en $C_1$-$C_4$.

Parmi les groupements azotés de la formule (II) ci-dessus, on peut citer notamment les radicaux amino, monoalkyl($C_1$-$C_4$)amino, dialkyl($C_1$-$C_4$)amino, trialkyl($C_1$-$C_4$)amino, monohydroxyalkyl($C_1$-$C_4$)amino, imidazolinium et ammonium.

Parmi les paraphénylènediamines de formule (II) ci-dessus, on peut plus particulièrement citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

Parmi les paraphénylènediamines de formule (II) ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide.

Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tinctoriales conformes à l'invention, on peut notamment citer les composés répondant à la formule (III) suivante, et leurs sels d'addition avec un acide :

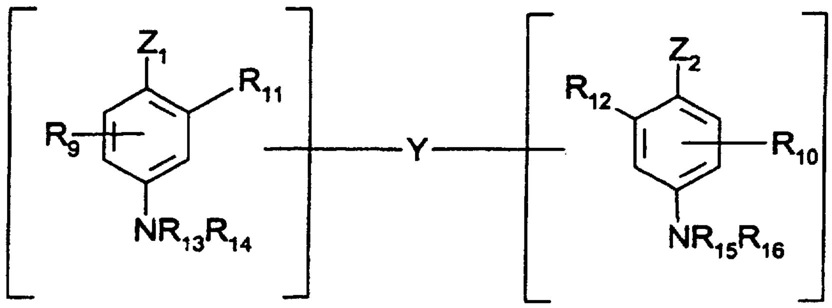 (III)

dans laquelle:

- $Z_1$ et $Z_2$, identiques ou différents, représentent un radical hydroxyle ou $-NH_2$ pouvant être substitué par un radical alkyle en $C_1$-$C_4$ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en $C_1$-$C_6$ ;
- $R_9$ et $R_{10}$ représentent un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$ ou un bras de liaison Y ;
- $R_{11}$, $R_{12}$, $R_{13}$, $R_{14}$, $R_{15}$ et $R_{16}$, identiques ou différents, représentent un atome d'hydrogène, un bras de liaison Y ou un radical alkyle en $C_1$-$C_4$ ;

étant entendu que les composés de formule (III) ne comportent qu'un seul bras de liaison Y par molécule.

Parmi les groupements azotés de la formule (III) ci-dessus, on peut citer notamment les radicaux amino, monoalkyl($C_1$-$C_4$)amino, dialkyl($C_1$-$C_4$)amino, trialkyl($C_1$-$C_4$)amino, monohydroxyalkyl($C_1$-$C_4$)amino, imidazolinium et ammonium.

Parmi les bases doubles de formules (III) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi ces bases doubles de formule (III), le N,N'-bis-(β-hydroxyéthyl) N,N'-bis-(4'-aminophényl) 1,3-diamino propanol, le 1,8-bis-(2,5-diaminophénoxy)-3,5-dioxaoctane ou l'un de leurs sels d'addition avec un acide sont particulièrement préférés.

Parmi les para-aminophénols utilisables à titre de bases d'oxydation dans les compositions tinctoriales conformes à l'invention, on peut notamment citer les composés de formule (IV) suivante, et leurs sels d'addition avec un acide :

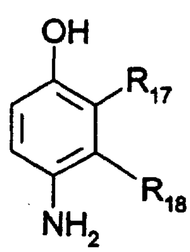 (IV)

dans laquelle :

- $R_{17}$ représente un atome d'hydrogène ou d'halogène, un radical alkyle en $C_1$-$C_4$, monohydroxyalkyle en $C_1$-$C_4$, alcoxy($C_1$-$C_4$)alkyle($C_1$-$C_4$), aminoalkyle en $C_1$-$C_4$ ou hydroxyalkyl($C_1$-$C_4$)aminoalkyle en $C_1$-$C_4$,
- $R_{18}$ représente un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxy($C_1$-$C_4$)alkyle($C_1$-$C_4$),

étant entendu qu'au moins un des radicaux $R_{17}$ et $R_{18}$ représente un atome d'hydrogène.

Parmi les para-aminophénols de formule (IV) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les orthoaminophénols utilisables à titre de bases d'oxydation dans les compositions tinctoriales conformes à l'invention, on peut plus particulièrement citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-Amino-pyrazolo[1,5-a]pyrimidin-7-yl)-(2-hydroxy-éthyl)-amino]-éthanol, le 2-[(7-Amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

La ou les bases d'oxydation représentent de préférence de 0,0005 à 12 % en poids environ du poids total de la composition tinctoriale conforme à l'invention, et encore plus préférentiellement de 0,005 à 6 % en poids environ de ce poids.

Le 1,3-bis-(β-hydroxyéthyl)amino 2-méthyl benzène et/ou le ou ses sels d'addition avec un acide utilisés à titre de premier coupleur selon l'invention, représentent de préférence de 0,001 à 10 % en poids environ du poids total de la composition tinctoriale et encore plus préférentiellement de 0,01 à 5 % en poids environ de ce poids.

Le ou les méta-aminophénols substitués de formules (I) et/ou (I bis) conformes à l'invention représentent de préférence de 0,0001 à 10 % en poids environ du poids total de la composition tinctoriale et encore plus préférentiellement de 0,005 à 5 % en poids environ de ce poids.

La composition tinctoriale conforme à l'invention peut en outre contenir un ou plusieurs coupleurs additionnels différents du 1,3-bis-(β-hydroxyéthyl)amino 2-méthyl benzène, des méta-aminophénols substitués de formules (I) et (I bis) et de leurs sels d'addition avec un acide et/ou un ou plusieurs colorants directs, notamment pour modifier les nuances ou les enrichir en reflets.

Parmi les coupleurs pouvant être présents à titre additionnel dans la composition tinctoriale conforme à l'invention, on peut notamment citer les métaphénylènediamines, les métadiphénols, les coupleurs hétérocycliques, et leurs sels d'addition avec un acide.

Lorsqu'ils sont présents ces coupleurs additionnels représentent de préférence de 0,0001 à 10 % en poids environ du poids total de la composition tin0ctoriale et encore plus préférentiellement de 0,005 à 5 % en poids environ

de ce poids.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

Le milieu approprié pour la teinture (ou support) de la composition tinctoriale conforme à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en $C_1$-$C_4$, tels que l'éthanol et l'isopropanol.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 12 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines, le 2-méthyl 2-amino propanol ainsi que leurs dérivés, les hydroxydes de sodium ou de potassium et les composés de formule (V) suivante :

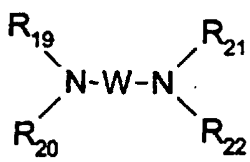

(V)

dans laquelle W est un reste propylène éventuellement substitué par un groupement hydroxyle ou un radical alkyle en $C_1$-$C_4$ ; $R_{19}$, $R_{20}$, $R_{21}$ et $R_{22}$, identiques ou différents, représentent un atome d'hydrogène, un radical alkyle en $C_1$-$C_4$ ou hydroxyalkyle en $C_1$-$C_4$.

La composition tinctoriale conforme à l'invention peut également renfermer divers adjuvants utilisés classiquement dans les compositions pour la teinture des cheveux.

Bien entendu, l'homme de l'art veillera à choisir ce ou ces éventuels composés complémentaires de manière telle que les propriétés avantageuses attachées intrinsèquement à la composition tinctoriale conforme à l'invention ne soient pas, ou substantiellement pas, altérées par la ou les adjonctions envisagées.

La composition tinctoriale conforme à l'invention peut se présenter sous des formes diverses, telles que sous forme de liquides, de crèmes, de gels, éventuellement pressurisés, ou sous toute autre forme appropriée pour réaliser une teinture des fibres kératiniques, et notamment des cheveux humains.

L'invention a également pour objet un procédé de teinture des fibres kératiniques, et en particulier des fibres kératiniques humaines telles que les cheveux mettant en oeuvre la composition tinctoriale telle que définie précédemment.

Selon ce procédé, on applique sur les fibres la composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre particulièrement préférée du procédé de teinture selon l'invention, on mélange, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant présent dans la composition oxydante telle que définie ci-dessus peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates, les peracides, les enzymes telles que les oxydoréductases à 2 électrons, les peroxydases et les laccases. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

**EXEMPLES**

**EXEMPLES DE TEINTURE 1 et 2 EN MILIEU ALCALIN**

On a préparé les compositions tinctoriales conformes à l'invention suivantes (teneurs en grammes) :

| EXEMPLE | 1 | 2 |
|---|---|---|
| Paraphénylènediamine (base d'oxydation) | 0,22 | - |
| Para-aminophénol (base d'oxydation) | - | 0,44 |
| 1,3-bis-(β-hydroxyéthyl)amino 2-méthyl benzène (premier coupleur) | 0,21 | 0,21 |
| 5-N-(β-hydroxyéthyl)amino 2-méthyl phénol, (coupleur de formule (I)) | 0,17 | - |
| 3-amino 2-chloro 6-méthyl phénol, (coupleur de formule (I bis)) | - | 0,32 |
| Support de teinture commun | (*) | (*) |
| Eau déminéralisée q.s.p. | 100 g | 100 g |

(*) : Support de teinture commun n°1 :
- Ethanol à 96° 18 g
- Métabisulfite de sodium en solution aqueuse à 35% 0,68 g
- Sel pentasodique de l'acide diéthylène triamino pentacétique 1,1 g
- Ammoniaque à 20% de $NH_3$ 10 g

Au moment de l'emploi, on a mélangé chacune des compositions tinctoriales décrites ci-dessus avec une quantité pondérale équivalente de peroxyde d'hydrogène à 20 volumes (6% en poids) présentant un pH d'environ 3.

Chaque mélange résultant présentait un pH d'environ 10 ± 0,2 et a été appliqué pendant 30 minutes sur des mèches de cheveux gris à 90 % de blancs permanentés.

Les cheveux ont ensuite été rincés à l'eau, lavés avec un shampooing standard, rincés à nouveau puis séchés.

Les cheveux ont été teints dans les nuances figurent dans le tableau ci-après :

| EXEMPLE | NUANCE OBTENUE |
|---|---|
| 1 | Violine irisé |
| 2 | Rouge cuivré |

**EXEMPLE 3 DE TEINTURE EN MILIEU ACIDE**

On a préparé la composition tinctoriale conforme à l'invention suivante :

- Paraphénylènediamine (base d'oxydation) 0,21 g
- 1,3-bis-(β-hydroxyéthyl)amino 2-méthyl benzène (premier coupleur) 0,21 g

- 5-N-(β-hydroxyéthyl)amino 2-méthyl phénol, (coupleur de formule (I)) 0,17 g
- Ethanol à 96° 18 g
- Métabisulfite de sodium en solution aqueuse à 35% 0,68 g
- Sel pentasodique de l'acide diéthylène triamino pentacétique 1,1 g
- Tampon $K_2HPO_4$ / $KH_2PO_4$ (1,5 M / 1 M) 10 g
- Eau déminéralisée qsp 100 g

Au moment de l'emploi, on a mélangé la composition tinctoriale décrites ci-dessus avec une quantité pondérale équivalente de peroxyde d'hydrogène à 20 volumes (6% en poids) présentant un pH d'environ 3.

Le mélange résultant présentait un pH d'environ 6,8 ± 0,2 et a été appliqué pendant 30 minutes sur des mèches de cheveux gris à 90 % de blancs permanentés.

Les cheveux ont ensuite été rincés à l'eau, lavés avec un shampooing standard, rincés à nouveau puis séchés.

Les cheveux ont été teints dans une nuance violet irisé.

## EXEMPLES 4 ET 5 COMPARATIFS

On a préparé les compositions tinctoriales suivantes (teneurs en grammes) :

| EXEMPLE | 4 | 5 (**) |
|---|---|---|
| Para-aminophénol (base d'oxydation) | 0,327 | 0,327 |
| 1,3-bis-(β-hydroxyéthyl)amino 2-méthyl benzène (premier coupleur) | 0,315 | 0,315 |
| 2-méthyl 5-N-(β-hydroxyéthyl)amino phénol (coupleur de formule (I) : 1,5 $10^{-3}$ mole) | 0,2505 | - |
| 2-méthyl 5-amino phénol (coupleur ne faisant pas partie de l'invention : 1,5 $10^{-3}$ mole) | - | 0,1845 |
| Support de teinture commun n°2 | (***) | (***) |
| Eau déminéralisée qsp | 100 g | 100 g |

(**) : exemple comparatif ne faisant pas partie de l'invention
Il est important de noter que chacune des compositions tinctoriales des exemples 5 et 6 ci-dessus renferme la même quantité molaire de coupleur additionnel, à savoir 1,5 $10^{-3}$ mole.

(***) : Support de teinture commun n°2 :
- Alcool oléique polyglycérolé à 2 moles de glycérol 4,0 g
- Alcool oléique polyglycérolé à 4 moles de glycérol à 78 % de matières actives (M.A.) 5,69 g M.A.
- Acide oléique 3,0 g
- Amine oléique à 2 moles d'oxyde d'éthylène vendue sous la dénomination commerciale ETHOMEEN O12 ® par la société AKZO 7,0 g
- Laurylamino succinamate de diéthylaminopropyle, sel de sodium à 55 % de M.A. 3,0 g M.A.
- Alcool oléique 5,0 g
- Diéthanolamide d'acide oléique 12,0 g
- Propylèneglycol 3,5 g
- Alcool éthylique 7,0 g
- Dipropylèneglycol 0,5 g
- Monométhyléther de propylèneglycol 9,0 g
- Métabisulfite de sodium à en solution aqueuse à 35 % de M.A. 0,455 g M.A.
- Acétate d'ammonium 0,8 g
- Antioxydant, séquestrant q.s.
- Parfum, conservateur q.s.
- Ammoniaque à 20 % de $NH_3$ 10,0 g

Au moment de l'emploi, on a mélangé chacune des compositions tinctoriales décrites ci-dessus avec une quantité pondérale équivalente de peroxyde d'hydrogène à 20 volumes (6% en poids) présentant un pH d'environ 3.

Chaque mélange résultant présentait un pH d'environ 10 ± 0,2 et a été appliqué pendant 30 minutes sur des mèches de cheveux gris permanentés à 90 % de blancs.

Les cheveux ont ensuite été rincés à l'eau, lavés avec un shampooing standard, rincés à nouveau puis séchés.

La couleur des mèches a été évaluée dans le système MUNSELL au moyen d'un spectrophotomètre MINOLTA CM 2002 ®.

Sur les mèches de cheveux ainsi teintes, il a été effectué un test de résistance à la permanente.

Pour ce faire, les mèches de cheveux ont été immergées pendant 10 minutes dans une solution de réducteur à raison de 2 g de la solution réductrice suivante par mèche de 1 g :

Solution réductrice :

- Acide thioglycolique 6,7 g
- Dithioglycolate de diammonium à 48 % dans l'eau 5,0 g
- Agent alcalinisant q.s. pH = 7,9
- Eau déminéralisée q.s.p. 100,0 g

Après rinçage, les mèches de cheveux ont été immergées pendant 5 minutes dans une solution oxydante (constituée par une solution de peroxyde d'hydrogène à 8 volumes et de pH 3) à raison de 2 g de solution de oxydante par mèche de 1g.

Les mèches ont ensuite été rincées à l'eau puis séchées pendant 1 heure à 60 °C.

La couleur des mèches de cheveux a été ensuite évaluée à nouveau dans le système MUNSELL au moyen d'un spectrophotomètre MINOLTA CM 2002 ®.

La différence entre la couleur de la mèche avant la permanente et la couleur de la mèche après la permanente a été calculée en appliquant la formule de NICKERSON :

$$\Delta E = 0,4\ Co\Delta H + 6\Delta V + 3\ \Delta C$$

telle que décrite par exemple dans "Couleur, Industrie et Technique" ; pages 14-17 ; vol. n° 5 ; 1978.

Dans cette formule, ΔE représente la différence de couleur entre deux mèches, ΔH, ΔV et ΔC représentent la variation en valeur absolue des paramètres H, V et C et Co représente la pureté de la mèche par rapport à laquelle on désire évaluer la différence de couleur, (avant le test de résistance à la permanente).

La dégradation de couleur (ΔE) est d'autant plus importante que le chiffre indiqué est élevé.

Les résultats sont donnés dans le tableau ci-dessous :

| EXEMPLE | Couleur des cheveux avant la permanente | Couleur des cheveux après la permanente | Dégradation de la couleur | | | |
|---|---|---|---|---|---|---|
| | | | ΔH | ΔV | ΔC | **ΔE** |
| **4** | 3.8 R 3.4 / 5.0 | 3.7 R 4.0 / 3.9 | 0.1 | 0.6 | 1.1 | **7.1** |
| **5(**)** | 4.7 R 3.2 / 4.8 | 3.3 R 4.1/ 3.5 | 1.4 | 0.9 | 1.3 | **12.0** |

(**) : Exemple ne faisant pas partie de l'invention.

Ces résultats montrent que la composition tinctoriale de l'exemple 4 conforme à l'invention et contenant l'association d'une base d'oxydation (para-aminophénol), du 1,3-bis-(β-hydroxyéthyl)amino 2-méthyl benzène à titre de premier coupleur, et du 2-méthyl 5-N-(β-hydroxyéthyl)amino phénol à titre de coupleur additionnel conforme à la formule (I), conduit à une coloration résistant beaucoup mieux à l'action de la permanente que la coloration obtenue en mettant en oeuvre la composition de l'exemple 5 ne faisant pas partie de l'invention car contenant, à la place du 2-méthyl 5-N-(β-hydroxyéthyl)amino phénol, le 2-méthyl 5-amino phénol tel que décrit par exemple dans la demande de brevet FR 2 681 860.

**EXEMPLES 6 ET 7 COMPARATIFS**

On a préparé les compositions tinctoriales suivantes (teneurs en grammes) :

| **EXEMPLE** | **6** | **7(**)** |
|---|---|---|
| Para-aminophénol (base d'oxydation) | 0,327 | 0,327 |
| 1,3-bis-(β-hydroxyéthyl)amino 2-méthyl benzène (premier coupleur) | 0,315 | 0,315 |
| 2-méthyl 5-N-(β-hydroxyéthyl)amino phénol (coupleur de formule (I) : 1,5 $10^{-3}$ mole) | 0,2505 | - |

(**) : exemple comparatif ne faisant pas partie de l'invention
Il est important de noter que chacune des compositions tinctoriales des exemples 5 et 6 ci-dessus renferme la même quantité molaire de coupleur additionnel, à savoir 1,5 $10^{-3}$ mole.

(suite)

| EXEMPLE | 6 | 7(**) |
|---|---|---|
| Méta-aminophénol (coupleur ne faisant pas partie de l'invention : 1,5 $10^{-3}$ mole) | - | 0,1635 |
| Support de teinture commun n°2 | (***) | (***) |
| Eau déminéralisée qsp | 100 g | 100 g |

(**) : exemple comparatif ne faisant pas partie de l'invention
Il est important de noter que chacune des compositions tinctoriales des exemples 5 et 6 ci-dessus renferme la même quantité molaire de coupleur additionnel, à savoir 1,5 $10^{-3}$ mole.

(***) : Support de teinture commun n°2 :
Il est identique à celui utilisé pour les exemples comparatifs 4 et 5 ci-dessus.

Au moment de l'emploi, on a mélangé chacune des compositions tinctoriales décrites ci-dessus avec une quantité pondérale équivalente de peroxyde d'hydrogène à 20 volumes (6% en poids) présentant un pH d'environ 3.

Chaque mélange résultant présentait un pH d'environ 10 ± 0,2 et a été appliqué pendant 30 minutes d'une part sur des mèches de cheveux gris naturels à 90 % de blancs, et d'autre part, sur des mèches de cheveux gris permanentés à 90 % de blancs.

Les cheveux ont ensuite été rincés à l'eau, lavés avec un shampooing standard, rincés à nouveau puis séchés.

La couleur des mèches a été évaluée dans le système MUNSELL au moyen d'un spectrophotomètre MINOLTA CM 2002 ®.

La différence de couleur entre les mèches de cheveux teints naturels et les mèches de cheveux teints permanentés correspondant à la sélectivité de la coloration a été calculée en appliquant la formule de NICKERSON :

$$\Delta E = 0{,}4\ C_o\ dH + 6\ dV + 3\ dC$$

telle que décrite par exemple dans "Journal of the Optical Society of America", vol.34, N°. 9, Sept 1944, pages 550-570.

Dans cette équation, ΔE représente la différence de couleur entre deux mèches, (dans le cas présent la sélectivité de la coloration), dH, dV, et dC représente la variation en valeur absolue des trois paramètres H, V, et C, Co représentant la saturation de la mèche par rapport à laquelle on veut évaluer la différence de couleur.

Plus la valeur de ΔE est importante, plus la différence de couleur entre les deux mèches est importante, et dans le cas présent, plus la sélectivité de la coloration est importante.

Les résultats figurent dans le tableau ci-après :

| Exemple | Couleur sur cheveux naturels | Couleur sur cheveux permanentés | Sélectivité de la coloration | | | |
|---|---|---|---|---|---|---|
| | | | dH | dV | dC | ΔE |
| **6** | 5.1 R 3.8 / 3.7 | 3.8 R 3.4 / 5.0 | 1.3 | 0.4 | 1.3 | **8.2** |
| **7 (**)** | 2.6 R 3.8 / 3.3 | 8.3 RP 3.2/4.9 | 4.3 | 0.6 | 1.6 | **14.1** |

Ces résultats montrent que la composition tinctoriale de l'exemple 6 conforme à l'invention et contenant l'association d'une base d'oxydation (para-aminophénol), du 1,3-bis-(β-hydroxyéthyl)amino 2-méthyl benzène à titre de premier coupleur, et du 2-méthyl 5-N-(β-hydroxyéthyl)amino phénol à titre de coupleur additionnel conforme à la formule (1), conduit à une coloration nettement moins sélective, (plus uniforme), que la coloration obtenue en mettant en oeuvre la composition de l'exemple 7 ne faisant pas partie de l'invention car contenant, à la place du 2-méthyl 5-N-(β-hydroxyéthyl)amino phénol, le méta-aminophénol tel que décrit par exemple dans la demande de brevet FR 2 681 860.

## Revendications

**1.** Composition pour la teinture d'oxydation des fibres kératiniques et en particulier des fibres kératiniques humaines telles que les cheveux, **caractérisée par le fait qu'**elle comprend, dans un milieu approprié pour la teinture :

- au moins une base d'oxydation,
- du 1,3-bis-(β-hydroxyéthyl)amino 2-méthyl benzène et/ou au moins l'un de ses sels d'addition avec un acide

à titre de premier coupleur ;

- et au moins un second coupleur choisi parmi les méta-aminophénols substitués de formules (I) et (I bis) ci-après et leurs sels d'addition avec un acide :

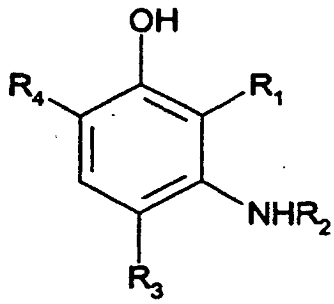

(I)

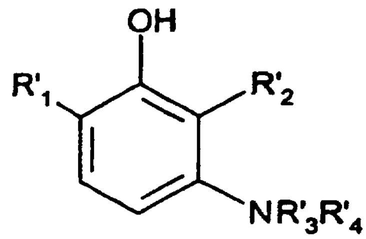

(I bis)

dans lesquelles :

- $R_1$ représente un atome d'hydrogène, un radical alkyle en $C_1$-$C_4$, monohydroxyalkyle en $C_1$-$C_4$, ou polyhydroxyalkyle en $C_2$-$C_4$ ;
- $R_2$ représente un atome d'hydrogène, un radical alkyle en $C_1$-$C_4$, monohydroxyalkyle en $C_1$-$C_4$ ou polyhydroxyalkyle en $C_2$-$C_4$ ;
- $R_3$ représente un atome d'hydrogène, un radical alkyle en $C_1$-$C_4$, alcoxy en $C_1$-$C_4$ ou un atome d'halogène choisi parmi le chlore, le brome, l'iode ou le fluor ;
- $R_4$ représente un atome d'hydrogène, un radical alkyle en $C_1$-$C_4$, alcoxy en $C_1$-$C_4$, monohydroxyalkyle en $C_1$-$C_4$, polyhydroxyalkyle en $C_2$-$C_4$, monohydroxyalcoxy en $C_1$-$C_4$ ou polyhydroxyalcoxy en $C_2$-$C_4$ ;
- $R'_1$ et $R'_2$, identiques ou différents, représentent un atome d'hydrogène ou d'halogène choisi parmi le chlore, le brome, l'iode ou le fluor, un radical alkyle en $C_1$-$C_4$, monohydroxyalkyle en $C_1$-$C_4$, polyhydroxyalkyle en $C_2$-$C_4$, alcoxy en $C_1$-$C_4$, monohydroxyalcoxy en $C_1$-$C_4$ ou polyhydroxyalcoxy en $C_2$-$C_4$ ;
- $R'_3$ et $R'_4$, identiques ou différents, représentent un atome d'hydrogène, un radical alkyle en $C_1$-$C_4$, monohydroxyalkyle en $C_1$-$C_4$, polyhydroxyalkyle en $C_2$-$C_4$ ou monoaminoalkyle en $C_1$-$C_4$ ;

étant entendu que :

- au moins un des radicaux $R_1$ à $R_4$ est différent d'un atome d'hydrogène ;
- au moins un des radicaux $R_1$, $R_3$ et $R_4$ représente un atome d'hydrogène ;
- au moins un des radicaux $R'_1$ et $R'_2$ représente un atome d'halogène ;
- et lorsque que $R_1$, $R_2$ et $R_3$ représentent simultanément un atome d'hydrogène, alors $R_4$ est différent d'un radical méthyle.

2. Composition selon la revendication 1, **caractérisée par le fait que** le ou les méta-aminophénols substitués de formules (I) ou (I bis) sont choisis parmi le 5-N-(β-hydroxyéthyl)amino 2-méthyl phénol, le 3-amino 2-chloro 6-méthyl phénol, le 5-amino 2-méthoxy phénol, le 5-amino 2-(β-hydroxyéthyloxy) phénol, le 5-N-(β-hydroxyéthyl)amino 4-méthoxy 2-méthyl phénol, le 5-amino 4-méthoxy 2-méthyl phénol, le 5-amino 4-chloro 2-méthyl phénol, le 5-amino 2,4-diméthoxy phénol, le 5-(γ-hydroxypropylamino) 2-méthyl phénol, le 3-amino 6-chloro phénol, le 3-amino 6-bromo phénol, le 3-(β-aminoéthyl)amino 6-chloro phénol, le 3-(β-hydroxyéthyl)amino 6-chloro phénol, et leurs sels d'addition avec un acide.

3. Composition selon la revendication 1 ou 2, **caractérisée par le fait que** la ou les bases d'oxydation sont choisies parmi les paraphénylènediamines, les bases doubles, les para-aminophénols, les ortho aminophénols et les bases d'oxydation hétérocycliques.

4. Composition selon la revendication 3, **caractérisée par le fait que** les paraphénylènediamines sont choisies parmi les composés de formule (II) suivante, et leurs sels d'addition avec un acide :

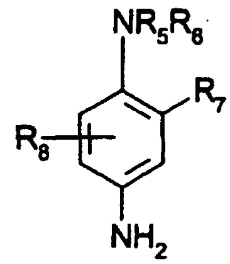

(II)

dans laquelle :

- $R_5$ représente un atome d'hydrogène, un radical alkyle en $C_1$-$C_4$, monohydroxyalkyle en $C_1$-$C_4$, polyhydroxyalkyle en $C_2$-$C_4$, alcoxy($C_1$-$C_4$)alkyle($C_1$-$C_4$), alkyle en $C_1$-$C_4$ substitué par un groupement azoté, phényle ou 4'-aminophényle ;
- $R_6$ représente un atome d'hydrogène, un radical alkyle en $C_1$-$C_4$, monohydroxyalkyle en $C_1$-$C_4$, polyhydroxyalkyle en $C_2$-$C_4$, alcoxy($C_1$-$C_4$)alkyle($C_1$-$C_4$) ou alkyle en $C_1$-$C_4$ substitué par un groupement azoté ;
- $R_7$ représente un atome d'hydrogène, un atome d'halogène tel qu'un atome de chlore, de brome, d'iode ou de fluor, un radical alkyle en $C_1$-$C_4$, monohydroxyalkyle en $C_1$-$C_4$, hydroxyalcoxy en $C_1$-$C_4$, acétylaminoalcoxy en $C_1$-$C_4$, mésylaminoalcoxy en $C_1$-$C_4$ ou carbamoylaminoalcoxy en $C_1$-$C_4$,
- $R_8$ représente un atome d'hydrogène, d'halogène ou un radical alkyle en $C_1$-$C_4$.

**5.** Composition selon la revendication 4, **caractérisée par le fait que** les paraphénylènediamine de formule (II) sont choisies parmi la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl) amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylènediamine, et leurs sels d'addition avec un acide.

**6.** Composition selon la revendication 3, **caractérisée par le fait que** les bases doubles sont choisies parmi les composés de formule (III) suivante, et leurs sels d'addition avec un acide :

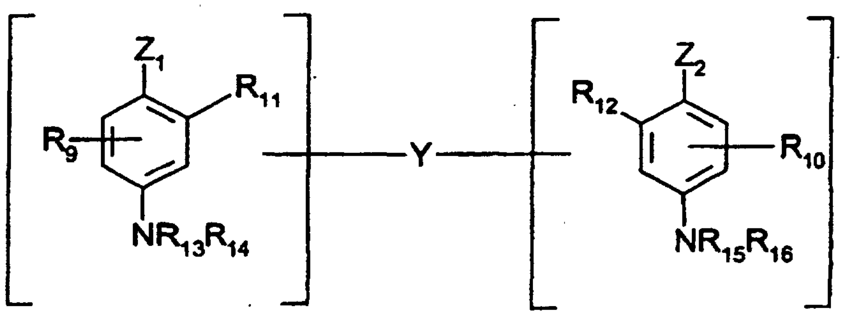

(III)

dans laquelle:

- $Z_1$ et $Z_2$, identiques ou différents, représentent un radical hydroxyle ou $-NH_2$ pouvant être substitué par un radical alkyle en $C_1$-$C_4$ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en $C_1$-$C_6$ ;

- $R_9$ et $R_{10}$ représentent un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$ ou un bras de liaison Y ;
- $R_{11}$, $R_{12}$, $R_{13}$, $R_{14}$, $R_{15}$ et $R_{16}$, identiques ou différents, représentent un atome d'hydrogène, un bras de liaison Y ou un radical alkyle en $C_1$-$C_4$ ;

étant entendu que les composés de formule (III) ne comportent qu'un seul bras de liaison Y par molécule.

**7.** Composition selon la revendication 6, **caractérisée par le fait que** les bases doubles de formule (III) sont choisies parmi le N,N'-bis-(β-hydroxyéthyl) N,N'-bis-(4'-aminophényl) 1,3-diamino propanol, la N,N'-bis-(β-hydroxyéthyl) N, N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylène diamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

**8.** Composition selon la revendication 3, **caractérisée par le fait que** para-aminophénols sont choisis parmi les composés de formule (IV) suivante, et leurs sels d'addition avec un acide :

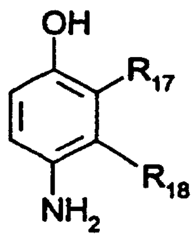

(IV)

dans laquelle :

- $R_{17}$ représente un atome d'hydrogène ou d'halogène, un radical alkyle en $C_1$-$C_4$, monohydroxyalkyle en $C_1$-$C_4$, alcoxy($C_1$-$C_4$)alkyle($C_1$-$C_4$), aminoalkyle en $C_1$-$C_4$ ou hydroxyalkyl($C_1$-$C_4$)aminoalkyle en $C_1$-$C_4$,
- $R_{18}$ représente un atome d'hydrogène ou d'halogène, un radical alkyle en $C_1$-$C_4$, monohydroxyalkyle en $C_1$-$C_4$, polyhydroxyalkyle en $C_2$-$C_4$, aminoalkyle en $C_1$-$C_4$, cyanoalkyle en $C_1$-$C_4$ ou alcoxy($C_1$-$C_4$)alkyle($C_1$-$C_4$),

étant entendu qu'au moins un des radicaux $R_{17}$ et $R_{18}$ représente un atome d'hydrogène.

**9.** Composition selon la revendication 8, **caractérisée par le fait que** les para-aminophénols de formule (IV) sont choisis parmi le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

**10.** Composition selon la revendication 3, **caractérisée par le fait que** les orthoaminophénols sont choisis parmi le 2-amino phénol, le 2-amino 5-méthyl phénol, le 2-amino 6-méthyl phénol, le 5-acétamido 2-amino phénol, et leurs sels d'addition avec un acide.

**11.** Composition selon la revendication 3, **caractérisée par le fait que** les bases d'oxydation hétérocycliques sont choisies parmi les dérivés pyridiniques, les dérivés pyrimidiniques, les dérivés pyrazoliques, et leurs sels d'addition avec un acide.

**12.** Composition selon l'une quelconque des revendications précédentes, **caractérisée par le fait que** la ou les bases d'oxydation représentent de 0,0005 à 12 % en poids du poids total de la composition tinctoriale.

**13.** Composition selon la revendication 12, **caractérisée par le fait que** la ou les bases d'oxydation représentent de 0,005 à 6 % en poids du poids total de la composition tinctoriale.

**14.** Composition selon l'une quelconque des revendications précédentes, **caractérisée par le fait que** le 1,3-bis-(β-hydroxyéthyl)amino 2-méthyl benzène et/ou le ou ses sels d'addition avec un acide représentent de 0,001 à 10

% en poids du poids total de la composition tinctoriale.

**15.** Composition selon la revendication 14, **caractérisée par le fait que** le 1,3-bis-(β-hydroxyéthyl)amino 2-méthyl benzène et/ou le ou ses sels d'addition avec un acide représentent de 0,01 à 5 % en poids du poids total de la composition tinctoriale.

**16.** Composition selon l'une quelconque des revendications précédentes, **caractérisée par le fait que** le ou les méta-aminophénols substitués de formules (I) et/ou (I bis) représentent de 0,0001 à 10 % en poids du poids total de la composition tinctoriale.

**17.** Composition selon la revendication 16, **caractérisée par le fait que** le ou les méta-aminophénols substitués de formule (I) et/ou (I bis) représentent de 0,005 à 5 % en poids du poids total de la composition tinctoriale.

**18.** Composition selon l'une quelconque des revendications précédentes, **caractérisée par le fait qu'**elle renferme un ou plusieurs coupleurs additionnels différents du 1,3-bis-(β-hydroxyéthyl)amino 2-méthyl benzène, des méta-aminophénols substitués de formules (I) et (I bis) tels que définis à la revendication 1 et de leurs sels d'addition avec un acide et/ou un ou plusieurs colorants directs.

**19.** Composition selon l'une quelconque des revendications précédentes, **caractérisée par le fait que** les sels d'addition avec un acide sont choisis parmi les chlorhydrates, les bromhydrates, les sulfates et les tartrates, les lactates et les acétates.

**20.** Procédé de teinture des fibres kératiniques et en particulier des fibres kératiniques humaines telles que les cheveux, **caractérisé par le fait qu'**on applique sur lesdites fibres au moins une composition tinctoriale telle que définie à l'une quelconque des revendications 1 à 19, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement.

**21.** Procédé selon la revendication 20, **caractérisé par le fait que** l'agent oxydant présent dans la composition oxydante est choisi parmi le peroxyde d'hydrogène, le peroxyde d'urée, les bromates de métaux alcalins, les persels tels que les perborates, les percarbonates et persulfates, les peracides, et les enzymes.

**22.** Dispositif à plusieurs compartiments, ou "kit" de teinture à plusieurs compartiments, dont un premier compartiment renferme une composition tinctoriale telle que définie à l'une quelconque des revendications 1 à 19 et un second compartiment renferme une composition oxydante.

**Patentansprüche**

**1.** Zusammensetzung zum oxidativen Färben von Keratinfasern und insbesondere menschlichen Keratinfasern, wie dem Haar, **dadurch gekennzeichnet, dass** sie in einem zum Färben geeigneten Medium enthält:

- mindestens eine Oxidationsbase,
- das 1,3-Bis(β-hydroxyethyl)amino-2-methyl-benzol und/oder mindestens ein Additionssalz dieser Verbindung mit einer Säure als ersten Kuppler, und
- mindestens einen zweiten Kuppler, der unter den substituierten m-Aminophenolen der folgenden Formeln (I) und (I bis) und deren Additionssalzen mit einer Säure ausgewählt ist:

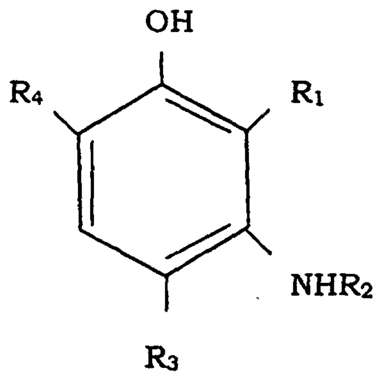
(I)

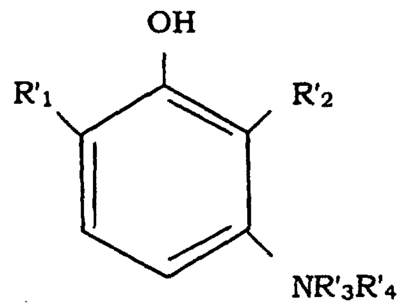
(Ibis)

worin bedeuten:

- $R_1$ Wasserstoff, $C_{1-4}$-Alkyl, $C_{1-4}$-Monohydroxyalkyl oder $C_{2-4}$-Polyhydroxyalkyl;
- $R_2$ Wasserstoff, $C_{1-4}$-Alkyl, $C_{1-4}$-Monohydroxyalkyl oder $C_{2-4}$-Polyhydroxyalkyl;
- $R_3$ Wasserstoff, $C_{1-4}$-Alkyl, $C_{1-4}$-Alkoxy oder ein Halogenatom, das unter Chlor, Brom, Iod oder Fluor ausgewählt ist;
- $R_4$ Wasserstoff, $C_{1-4}$-Alkyl, $C_{1-4}$-Alkoxy, $C_{1-4}$-Monohydroxyalkyl, $C_{2-4}$-Polyhydroxyalkyl, $C_{1-4}$-Monohydroxyalkoxy oder $C_{2-4}$-Polyhydroxyalkoxy;
- $R'_1$ und $R'_2$, die gleich oder verschieden sind, Wasserstoff, ein Halogenatom, das unter Chlor, Brom, Iod oder Fluor ausgewählt ist, $C_{1-4}$-Alkyl, $C_{1-4}$-Monohydroxyalkyl, $C_{2-4}$-Polyhydroxyalkyl, $C_{1-4}$-Alkoxy, $C_{1-4}$-Monohydroxyalkoxy oder $C_{2-4}$-Polyhydroxyalkoxy;
- $R'_3$ und $R'_4$, die gleich oder verschieden sind, Wasserstoff, $C_{1-4}$-Alkyl, $C_{1-4}$-Monohydroxyalkyl, $C_{2-4}$-Polyhydroxyalkyl oder $C_{1-4}$-Monoaminoalkyl;

mit der Maßgabe, dass

- mindestens eine der Gruppen $R_1$ bis $R_4$ von Wasserstoff verschieden ist;
- mindestens eine der Gruppen $R_1$, $R_3$ und $R_4$ Wasserstoff bedeutet;
- mindestens eine der Gruppen $R'_1$ und $R'_2$ ein Halogenatom bedeutet; und
- die Gruppe $R_4$ von Methyl verschieden ist, wenn die Gruppen $R_1$, $R_2$ und $R_3$ gleichzeitig Wasserstoff bedeuten.

**2.** Zusammensetzung nach Anspruch 1, **dadurch gekennzeichnet, dass** das oder die substituierte(n) m-Aminophenol(e) der oben angegebenen Formeln (I) und (Ibis) unter 5-N-(β-Hydroxyethyl)amino-2-methyl-phenol, 3-Amino-2-chlor-6-methyl-phenol, 5-Amino-2-methoxyphenol, 5-Amino-2-(β-hydroxyethyloxy)-phenol, 5-N-(β-Hydroxyethyl)amino-4-methoxy-2-methyl-phenol, 5-Amino-4-methoxy-2-methyl-phenol, 5-Amino-4-chlor-2-methyl-phenol, 5-Amino-2,4-dimethoxy-phenol, 5-(γ-Hydroxypropylamino)-2-methylphenol, 3-Amino-6-chlor-phenol, 3-Amino-6-brom-phenol, 3-(β-Aminoethyl)amino-6-chlor-phenol, 3-(β-Hydroxyethyl)amino-6-chlorphenol und den Additionssalzen dieser Verbindungen mit einer Säure ausgewählt sind.

**3.** Zusammensetzung nach einem der Ansprüche 1 oder 2, **dadurch gekennzeichnet, dass** die Oxidationsbase(n) unter den p-Phenylendiaminen, Doppelbasen, p-Aminophenolen, o-Aminophenolen und den heterocyclischen Oxidationsbasen ausgewählt sind.

**4.** Zusammensetzung nach Anspruch 3, **dadurch gekennzeichnet, dass** die p-Phenylendiamine unter den Verbindungen der folgenden Formel (II) und deren Additionssalzen mit einer Säure ausgewählt sind:

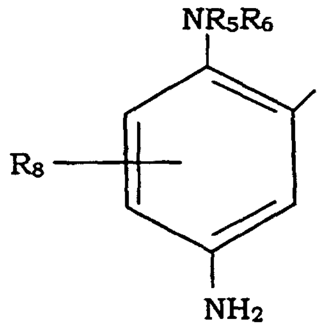

(II),

worin bedeuten: .

- $R_5$ Wasserstoff, $C_{1-4}$-Alkyl, $C_{1-4}$-Monohydroxyalkyl, $C_{2-4}$-Polyhydroxyalkyl, $C_{1-4}$-Alkoxy-$C_{1-4}$-alkyl, eine mit einer stickstoffhaltigen Gruppe substituierte $C_{1-4}$-Alkylgruppe, Phenyl oder 4'-Aminophenyl,
- $R_6$ Wasserstoff, $C_{1-4}$-Alkyl, $C_{1-4}$-Monohydroxyalkyl, $C_{2-4}$-Polyhydroxyalkyl, $C_{1-4}$-Alkoxy-$C_{1-4}$-alkyl oder eine mit einer stickstoffhaltigen Gruppe substituierte $C_{1-4}$-Alkylgruppe,
- $R_7$ Wasserstoff, Halogen, wie Chlor, Brom, Iod oder Fluor, $C_{1-4}$-Alkyl, $C_{1-4}$-Monohydroxyalkyl, $C_{1-4}$-Hydroxyalkoxy, $C_{1-4}$-Acetylaminoalkoxy, $C_{1-4}$-Mesylaminoalkoxy oder $C_{1-4}$-Carbamoylaminoalkoxy, und
- $R_8$ Wasserstoff, Halogen oder $C_{1-4}$-Alkyl.

**5.** Zusammensetzung nach Anspruch 4, **dadurch gekennzeichnet, dass** die p-Phenylendiamine der Formel (II) unter p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-N,N-diethyl-3-methyl-anilin, N,N-Bis(β-hydroxyethyl)-p-phenylendiamin, 4-N,N-Bis(β-hydroxyethyl)amino-2-methyl-anilin, 4-N,N-Bis(β-hydroxyethyl)amino-2-chlor-anilin, 2-β-Hydroxyethyl-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl, β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-β-Hydroxyethyloxy-p-phenylendiamin, 2-β-Acetylaminoethyloxy-p-phenylendiamin, N-(β-Methoxyethyl)-p-phenylendiamin und deren Additionssalzen mit einer Säure ausgewählt sind.

**6.** Zusammensetzung nach Anspruch 3, **dadurch gekennzeichnet, dass** die Doppelbasen unter den Verbindungen der folgenden Formel (III) und deren Additionssalze mit einer Säure ausgewählt sind:

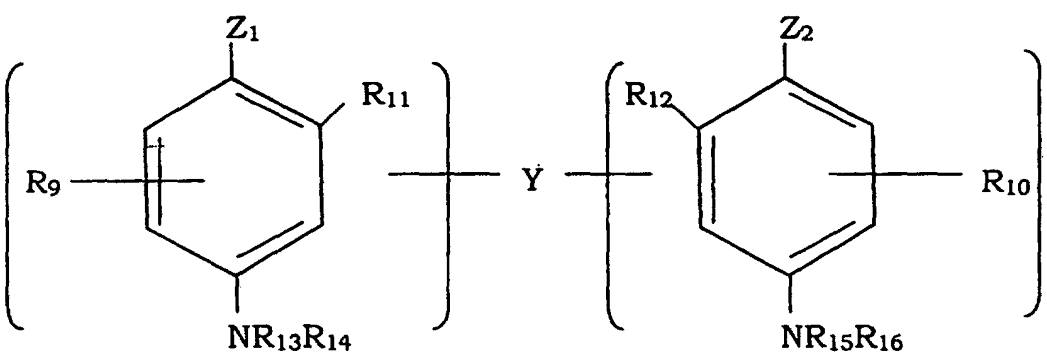

(III)

worin bedeuten:

- $Z_1$ und $Z_2$, die identisch oder voneinander verschieden sind, eine Hydroxygruppe oder eine $NH_2$-Gruppe, die mit $C_{1-4}$-Alkyl oder einer Verbindungsgruppe Y substituiert sein kann,
- die Verbindungsgruppe Y eine geradkettige oder verzweigte Alkylengruppe mit 1 bis 14 Kohlenstoffatomen,

die durch eine oder mehrere stickstoffhaltige Gruppen und/oder ein oder mehrere Heteroatome, wie Sauerstoff, Schwefel oder Stickstoff, unterbrochen oder abgeschlossen werden kann und gegebenenfalls mit einer oder mehreren Hydroxy- oder $C_{1-6}$-Alkoxygruppen substituiert sein kann,

- $R_9$ und $R_{10}$ Wasserstoff, Halogen, $C_{1-4}$-Alkyl, $C_{1-4}$-Monohydroxyalkyl, $C_{2-4}$-Polyhydroxyalkyl, $C_{1-4}$-Aminoalkyl oder eine Verbindungsgruppe Y,
- $R_{11}$, $R_{12}$, $R_{13}$, $R_{14}$, $R_{15}$ und $R_{16}$, die gleich oder verschieden sind, Wasserstoff, eine Verbindungsgruppe Y oder $C_{1-4}$-Alkyl,

mit der Maßgabe, dass die Verbindungen der Formel (III) nur eine Verbindungsgruppe Y pro Molekül aufweisen.

**7.** Zusammensetzung nach Anspruch 6, **dadurch gekennzeichnet, dass** die Doppelbasen der Formel (III) unter N, N'-Bis(β-hydroxyethyl)-N,N'-bis-(4'-aminophenyl)-1,3-diaminopropanol, N,N'-Bis(β-hydroxyethyl)-N,N'-bis(4'-aminophenyl)-ethyleridiamin, N,N'-Bis(4-aminophenyl)-tetramethylendiamin, N,N'-Bis(β-hydroxyethyl)-N,N'-bis (4-aminophenyl)-tetramethylendiamin, N,N'-Bis(4-methylaminophenyl)-tetramethylendiamin, N,N'-Bis(ethyl)-N, N'-bis-(4'-amino, 3'-methylphenyl)-ethylendiamin, 1,8-Bis(2,5-diamino-phenoxy)-3,5-dioxaoctan und deren Additionssalzen mit einer Säure ausgewählt sind.

**8.** Zusammensetzung nach Anspruch 3, **dadurch gekennzeichnet, dass** die p-Aminophenole unter den Verbindungen der folgenden Formel (IV) und deren Additionssalzen mit einer Säure ausgewählt sind:

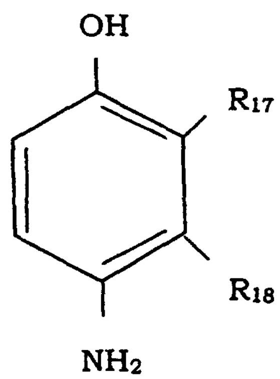

(IV)

worin bedeuten:

- $R_{17}$ Wasserstoff, Halogen, $C_{1-4}$-Alkyl, $C_{1-4}$-Monohydroxyalkyl, $C_{1-4}$-Alkoxy-$C_{1-4}$-alkyl, $C_{1-4}$-Aminoalkyl oder $C_{1-4}$-Hydroxyalkyl-$C_{1-4}$-aminoalkyl,
- $R_{18}$ Wasserstoff, Halogen, $C_{1-4}$-Alkyl, $C_{1-4}$-Monohydroxyalkyl, $C_{2-4}$-Polyhydroxyalkyl, $C_{1-4}$-Aminoalkyl, $C_{1-4}$-Cyanoalkyl oder $C_{1-4}$-Alkoxy-$C_{1-4}$-alkyl,

mit der Maßgabe, dass mindestens eine der Gruppen $R_{17}$ oder $R_{18}$ Wasserstoff bedeutet.

**9.** Zusammensetzung nach Anspruch 8, **dadurch gekennzeichnet, dass** die p-Aminophenole der Formel (IV) unter p-Aminophenol, 4-Amino-3-methyl-phenol, 4-Amino-3-fluor-phenol, 4-Amino-3-hydroxymethyl-phenol, 4-Amino-2-methyl-phenol, 4-Amino-2-hydroxymethyl-phenol, 4-Amino-2-methoxymethyl-phenol, 4-Amino-2-aminomethyl-phenol, 4-Amino-2-(β-hydroxyethylaminomethyl)-phenol, 4-Amino-2-fluor-phenol und deren Additionssalzen mit einer Säure ausgewählt sind.

**10.** Zusammensetzung nach Anspruch 3, **dadurch gekennzeichnet, dass** die o-Aminophenole unter 2-Aminophenol, 2-Amino-5-methylphenol, 2-Amino-6-methyl-phenol, 5-Acetamido-2-amino-phenol und deren Additionssalzen mit einer Säure ausgewählt sind.

**11.** Zusammensetzung nach Anspruch 3, **dadurch gekennzeichnet, dass** die heterocyclischen Oxidationsbasen unter den Pyridinderivaten, Pyrimidinderivaten, Pyrazolderivaten und deren Additionssalzen mit einer Säure ausgewählt sind.

**12.** Zusammensetzung nach einem der vorhergehenden Ansprüche, **dadurch gekennzeichnet, dass** die Oxidationsbase(n) 0,0005 bis 12 Gew.-% des Gesamtgewichts der Farbmittelzusammensetzung ausmachen.

**13.** Zusammensetzung nach Anspruch 12, **dadurch gekennzeichnet, dass** die Oxidationsbase(n) 0,005 bis 6 Gew.-% des Gesamtgewichts der Farbmittelzusammensetzung ausmachen.

**14.** Zusammensetzung nach einem der vorhergehenden Ansprüche, **dadurch gekennzeichnet, dass** das 1,3-Bis(β-hydroxyethyl)amino-2-methyl-benzol und/oder sein(e) Additionssalz(e) mit einer Säure 0,001 bis 10 Gew.-% des Gesamtgewichts der Farbmittelzusammensetzung ausmachen.

**15.** Zusammensetzung nach Anspruch 14, **dadurch gekennzeichnet, dass** das 1,3-Bis(β-hydroxyethyl)amino-2-methyl-benzol und/oder sein(e) Additionssalz(e) mit einer Säure 0,01 bis 5 Gew.-% des Gesamtgewichts der Farbmittelzusammensetzung ausmachen.

**16.** Zusammensetzung nach einem der vorhergehenden Ansprüche, **dadurch gekennzeichnet, dass** das oder die substituierte(n) m-Aminophenol(e) der Formel (I) und/oder (Ibis) 0,0001 bis 10 Gew.-% des Gesamtgewichts der Farbmittelzusammensetzung ausmachen.

**17.** Zusammensetzung nach Anspruch 16, **dadurch gekennzeichnet, dass** das das oder die substituierte(n) m-Aminophenol(e) der Formel (I) und/oder (Ibis) 0,005 bis 5 Gew.-% des Gesamtgewichts der Farbmittelzusammensetzung ausmachen.

**18.** Zusammensetzung nach einem der vorhergehenden Ansprüche, **dadurch gekennzeichnet, dass** sie einen oder mehrere zusätzliche Kuppler, die von dem 1,3-Bis(β-hydroxyethyl)amino-2-methylbenzol, den substituierten m-Aminophenolen der Formeln (I) und (Ibis) nach Anspruch 1 und deren Additionssalzen mit einer Säure verschieden sind, und/oder einen oder mehrer Direktfarbstoffe enthält.

**19.** Zusammensetzung nach einem der vorhergehenden Ansprüche, **dadurch gekennzeichnet, dass** die Additionssalze mit einer Säure unter den Hydrochloriden, Hydrobromiden, Sulfaten, Tartraten, Lactaten und Acetaten ausgewählt sind.

**20.** Verfahren zum Färben von Keratinfasern und insbesondere menschlichen Keratinfasern, wie dem Haar, **dadurch gekennzeichnet, dass** auf die Fasern mindestens eine Farbmittelzusammensetzung nach einem der Ansprüche 1 bis 19 aufgebracht wird und die Farbe bei einem sauren, neutralen oder alkalischen pH-Wert mit einem Oxidationsmittel entwickelt wird, das unmittelbar bei der Anwendung zu der Farbmittelzusammensetzung gegeben wird oder das in einer oxidierenden Zusammensetzung vorliegt, die gleichzeitig dierenden Zusammensetzung vorliegt, die gleichzeitig oder getrennt davon anschließend aufgebracht wird.

**21.** Verfahren nach Anspruch 20, **dadurch gekennzeichnet, dass** das in der oxidierenden Zusammensetzung vorliegende Oxidationsmittel unter Wasserstoffperoxid, Harnstoffperoxid, Alkalimetallbromaten, Salzen von Persäuren, wie Perboraten, Perkarbonaten und Persulfaten, Persäuren und Enzymen ausgewählt ist.

**22.** Vorrichtung mit mehreren Abteilungen oder "Kit" zum Färben mit mehreren Abteilungen, **dadurch gekennzeichnet, dass** eine erste Abteilung die in den Ansprüchen 1 bis 19 definierte Farbmittelzusammensetzung und eine zweite Abteilung eine oxidierende Zusammensetzung enthält.

**Claims**

**1.** Composition for the oxidation dyeing of keratin fibres, and in particular human keratin fibres such as the hair, **characterized in that** it comprises, in a medium which is suitable for dyeing:

- at least one oxidation base,
- 1,3-bis(β-hydroxyethyl)amino-2-methylbenzene and/or at least one of the addition salts thereof with an acid, as first coupler;
- and at least one second coupler chosen from the substituted meta-aminophenols of formulae (I) and (Ia) below, and the addition salts thereof with an acid:

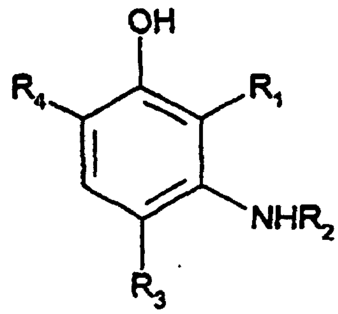

(I)

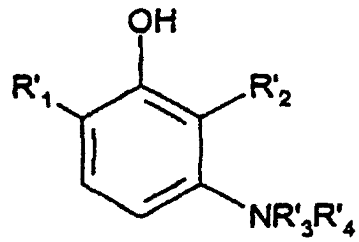

(Ia)

in which:

- $R_1$ represents a hydrogen atom, a $C_1$-$C_4$ alkyl radical, a $C_1$-$C_4$ monohydroxyalkyl radical or a $C_2$-$C_4$ polyhydroxyalkyl radical;
- $R_2$ represents a hydrogen atom, a $C_1$-$C_4$ alkyl radical, a $C_1$-$C_4$ monohydroxyalkyl radical or a $C_2$-$C_4$ polyhydroxyalkyl radical;
- $R_3$ represents a hydrogen atom, a $C_1$-$C_4$ alkyl radical, a $C_1$-$C_4$ alkoxy radical or a halogen atom chosen from chlorine, bromine, iodine and fluorine;
- $R_4$ represents a hydrogen atom, a $C_1$-$C_4$ alkyl radical, a $C_1$-$C_4$ alkoxy radical, a $C_1$-$C_4$ monohydroxyalkyl radical, a $C_2$-$C_4$ polyhydroxyalkyl radical, a $C_1$-$C_4$ monohydroxyalkoxy radical or a $C_2$-$C_4$ polyhydroxyalkoxy radical;
- $R'_1$ and $R'_2$, which may be identical or different, represent a hydrogen atom or a halogen atom chosen from chlorine, bromine, iodine and fluorine, a $C_1$-$C_4$ alkyl radical, a $C_1$-$C_4$ monohydroxyalkyl radical, a $C_2$-$C_4$ polyhydroxyalkyl radical, a $C_1$-$C_4$ alkoxy radical, a $C_1$-$C_4$ monohydroxyalkoxy radical or a $C_2$-$C_4$ polyhydroxyalkoxy radical;
- $R'_3$ and $R'_4$, which may be identical or different, represent a hydrogen atom, a $C_1$-$C_4$ alkyl radical, a $C_1$-$C_4$ monohydroxyalkyl radical, a $C_2$-$C_4$ polyhydroxyalkyl radical or a $C_1$-$C_4$ monoaminoalkyl radical;

it being understood that:

- at least one of the radicals $R_1$ to $R_4$ is other than a hydrogen atom;
- at least one of the radicals $R_1$, $R_3$ and $R_4$ represents a hydrogen atom;
- at least one of the radicals $R'_1$ and $R'_2$ represents a halogen atom;
- and when $R_1$, $R_2$ and $R_3$ simultaneously represent a hydrogen atom, then $R_4$ is other than a methyl radical.

**2.** Composition according to Claim 1, **characterized in that** the substituted meta-aminophenol(s) of formula (I) or (Ia) is (are) chosen from 5-N-(β-hydroxyethyl)amino-2-methylphenol, 3-amino-2-chloro-6-methylphenol, 5-amino-2-methoxyphenol, 5-amino-2-(β-hydroxyethyloxy)phenol, 5-N-(β-hydroxyethyl)amino-4-methoxy-2-methylphenol, 5-amino-4-methoxy-2-methylphenol, 5-amino-4-chloro-2-methylphenol, 5-amino-2,4-dimethoxyphenol, 5-(γ-hydroxypropylamino)-2-methylphenol, 3-amino-6-chlorophenol, 3-amino-6-bromophenol, 3-(β-aminoethyl)amino-6-chlorophenol and 3-(β-hydroxyethyl)amino-6-chlorophenol, and the addition salts thereof with an acid.

**3.** Composition according to Claim 1 or 2, **characterized in that** the oxidation base(s) is (are) chosen from para-phenylenediamines, double bases, para-aminophenols, ortho-aminophenols and heterocyclic oxidation bases.

**4.** Composition according to Claim 3, **characterized in that** the para-phenylenediamines are chosen from the compounds of formula (II) below, and the addition salts thereof with an acid:

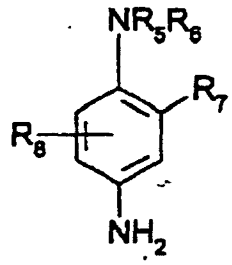

(II)

in which:

- $R_5$ represents a hydrogen atom, a $C_1$-$C_4$ alkyl radical, a $C_1$-$C_4$ monohydroxyalkyl radical, a $C_2$-$C_4$ polyhydroxyalkyl radical, a ($C_1$-$C_4$)alkoxy($C_1$-$C_4$)alkyl radical or a $C_1$-$C_4$ alkyl radical substituted with a nitrogenous, phenyl or 4'-aminophenyl group;
- $R_6$ represents a hydrogen atom, a $C_1$-$C_4$ alkyl radical, a $C_1$-$C_4$ monohydroxyalkyl radical, a $C_2$-$C_4$ polyhydroxyalkyl radical, a ($C_1$-$C_4$)alkoxy($C_1$-$C_4$)alkyl radical or a $C_1$-$C_4$ alkyl radical substituted with a nitrogenous group;
- $R_7$ represents a hydrogen atom, a halogen atom such as a chlorine, bromine, iodine or fluorine atom, a $C_1$-$C_4$ alkyl radical, a $C_1$-$C_4$ monohydroxyalkyl radical, a $C_1$-$C_4$ hydroxyalkoxy radical, a $C_1$-$C_4$ acetylaminoalkoxy radical, a $C_1$-$C_4$ mesylaminoalkoxy radical or a $C_1$-$C_4$ carbamoylaminoalkoxy radical,
- $R_8$ represents a hydrogen or halogen atom or a $C_1$-$C_4$ alkyl radical.

**5.** Composition according to Claim 4, **characterized in that** the para-phenylenediamines of formula (II) are chosen from para-phenylenediamine, para-tolylenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(β-hydroxyethyl)-para-phenylenediamine, 4-N,N-bis(β-hydroxyethyl)amino-2-methylaniline, 4-N,N-bis(β-hydroxyethyl)amino-2-chloroaniline, 2-β-hydroxyethyl-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N-ethyl-N-(β-hydroxyethyl)-para-phenylenediamine, N-(β,γ-dihydroxypropyl)-para-phenylenediamine, N-(4'-aminophenyl)-para-phenylenediamine, N-phenyl-para-phenylenediamine, 2-β-hydroxyethyloxy-para-phenylenediamine, 2-β-acetylaminoethyloxy-para-phenylenediamine and N-(β-methoxyethyl)-para-phenylenediamine, and the addition salts thereof with an acid.

**6.** Composition according to Claim 3, **characterized in that** the double bases are chosen from the compounds of formula (III) below, and the addition salts thereof with an acid:

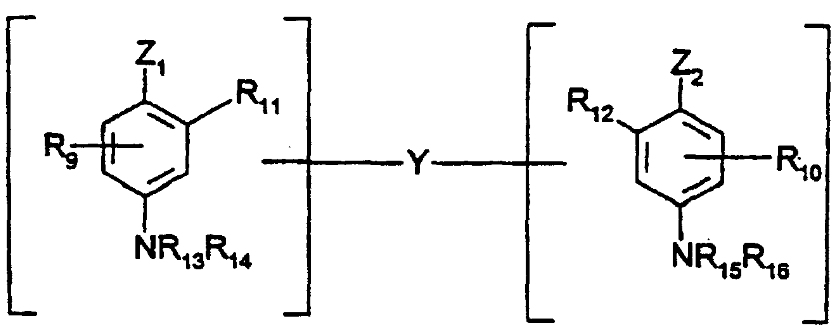

(III)

in which:

- $Z_1$ and $Z_2$, which may be identical or different, represent a hydroxyl or $-NH_2$ radical which can be substituted with a $C_1$-$C_4$ alkyl radical or with a linker arm Y;

- the linker arm Y represents a linear or branched alkylene chain comprising from 1 to 14 carbon atoms, which can be interrupted or terminated with one or more nitrogenous groups and/or with one or more hetero atoms such as oxygen, sulphur or nitrogen atoms, and optionally substituted with one or more hydroxyl or $C_1$-$C_6$ alkoxy radicals;
- $R_9$ and $R_{10}$ represent a hydrogen or halogen atom, a $C_1$-$C_4$ alkyl radical, a $C_1$-$C_4$ monohydroxyalkyl radical, a $C_2$-$C_4$ polyhydroxyalkyl radical, a $C_1$-$C_4$ aminoalkyl radical or a linker arm Y;
- $R_{11}$, $R_{12}$, $R_{13}$, $R_{14}$, $R_{15}$ and $R_{16}$, which may be identical or different, represent a hydrogen atom, a linker arm Y or a $C_1$-$C_4$ alkyl radical;

it being understood that the compounds of formula (III) comprise only one linker arm Y per molecule.

7. Composition according to Claim 6, **characterized in that** the double bases of formula (III) are chosen from N,N'-bis(β-hydroxyethyl)-N,-N'-bis(4'-aminophenyl)-1,3-diaminopropanol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis (4-aminophenyl)tetramethylenediamine, N,N'-bis(4-methylaminophenyl)tetramethylenediamine, N,N'-bis(ethyl) -N,N'-bis(4'-amino-3'-methylphenyl)ethylenediamine and 1,8-bis(2,5-diaminophenoxy)-3,5-dioxaoctane, and the addition salts thereof with an acid.

8. Composition according to Claim 3, **characterized in that** the para-aminophenols are chosen from the compounds of formula (IV) below, and the addition salts thereof with an acid:

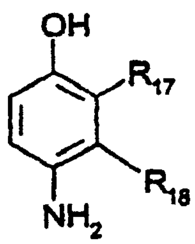

(IV)

in which:

- $R_{17}$ represents a hydrogen or halogen atom, a $C_1$-$C_4$ alkyl radical, a $C_1$-$C_4$ monohydroxyalkyl radical, a ($C_1$-$C_4$) alkoxy($C_1$-$C_4$)alkyl radical, a $C_1$-$C_4$ aminoalkyl radical or a hydroxy ($C_1$-$C_4$) alkylamino ($C_1$-$C_4$) alkyl radical,
- $R_{18}$ represents a hydrogen or halogen atom, a $C_1$-$C_4$ alkyl radical, a $C_1$-$C_4$ monohydroxyalkyl radical, a $C_2$-$C_4$ polyhydroxyalkyl radical, a $C_1$-$C_4$ aminoalkyl radical, a cyano($C_1$-$C_4$)alkyl radical or a ($C_1$-$C_4$)alkoxy($C_1$-$C_4$) alkyl radical,

it being understood that at least one of the radicals $R_{17}$ and $R_{18}$ represents a hydrogen atom.

9. Composition according to Claim 8, **characterized in that** the para-aminophenols of formula (IV) are chosen from para-aminophenol, 4-amino-3-methylphenol, 4-amino-3-fluorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxyethylaminomethyl)phenol and 4-amino-2-fluorophenol, and the addition salts thereof with an acid.

10. Composition according to Claim 3, **characterized in that** the ortho-aminophenols are chosen from 2-aminophenol, 2-amino-5-methylphenol, 2-amino-6-methylphenol and 5-acetamido-2-aminophenol, and the addition salts thereof with an acid.

11. Composition according to Claim 3, **characterized in that** the heterocyclic oxidation bases are chosen from pyridine derivatives, pyrimidine derivatives and pyrazole derivatives, and the addition salts thereof with an acid.

12. Composition according to any one of the preceding claims, **characterized in that** the oxidation base(s) represent (s) from 0.0005 to 12% by weight relative to the total weight of the dye composition.

**13.** Composition according to Claim 12, **characterized in that** the oxidation base(s) represent(s) from 0.005 to 6% by weight relative to the total weight of the dye composition.

**14.** Composition according to any one of the preceding claims, **characterized in that** the 1,3-bis(β-hydroxyethyl) amino-2-methylbenzene and/or the addition salt(s) thereof with an acid represent(s) from 0.001 to 10% by weight relative to the total weight of the dye composition.

**15.** Composition according to Claim 14, **characterized in that** the 1,3-bis(β-hydroxyethyl)amino-2-methylbenzene and/or the addition salt(s) thereof with an acid represent(s) from 0.01 to 5% by weight relative to the total weight of the dye composition.

**16.** Composition according to any one of the preceding claims, **characterized in that** the substituted meta-aminophenol(s) of formulae (I) and/or (Ia) represent(s) from 0.0001 to 10% by weight relative to the total weight of the dye composition.

**17.** Composition according to Claim 16, **characterized in that** the substituted meta-aminophenol(s) of formulae (I) and/or (Ia) represent(s) from 0.005 to 5% by weight relative to the total weight of the dye composition.

**18.** Composition according to any one of the preceding claims, **characterized in that** it contains one or more additional couplers other than 1,3-bis(β-hydroxyethyl)amino-2-methylbenzene, substituted meta-aminophenols of formulae (I) and (Ia) as defined in Claim 1 and the addition salts thereof with an acid, and/or one or more direct dyes.

**19.** Composition according to any one of the preceding claims, **characterized in that** the addition salts with an acid are chosen from the hydrochlorides, hydrobromides, sulphates, tartrates, lactates and acetates.

**20.** Process for dyeing keratin fibres, and in particular human keratin fibres such as the hair, **characterized in that** at least one dye composition as defined in any one of Claims 1 to 19 is applied to the said fibres and the colour is developed at acidic, neutral or alkaline pH using an oxidizing agent which is added to the dye composition just at the time of use, or which is present in an oxidizing composition that is applied simultaneously or sequentially.

**21.** Process according to Claim 20, **characterized in that** the oxidizing agent present in the oxidizing composition is chosen from hydrogen peroxide, urea peroxide, alkali metal bromates, persalts such as perborates, percarbonates and persulphates, peracids and enzymes.

**22.** Multi-compartment dyeing device or multicompartment dyeing "kit", a first compartment of which contains a dye composition as defined in any one of Claims 1 to 19 and a second compartment of which contains an oxidizing composition.